(19) Europäisches Patentamt
European Patent Office
Office européen des brevets

(11) **EP 4 174 721 A1**

(12) **EUROPEAN PATENT APPLICATION**

(43) Date of publication:
**03.05.2023 Bulletin 2023/18**

(21) Application number: **21205810.1**

(22) Date of filing: **01.11.2021**

(51) International Patent Classification (IPC):
*G06N 3/04* (2023.01)          *G06N 3/08* (2023.01)
*G06N 20/00* (2019.01)         *G06K 9/62* (2022.01)

(52) Cooperative Patent Classification (CPC):
**G06N 20/00;** G06N 3/045; G06N 5/01

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SUSAIYAH, Allmin Pradhap Singh**
**Eindhoven (NL)**
• **PATIL, Ravindra**
**Eindhoven (NL)**
• **ANAND, Shreya**
**Eindhoven (NL)**
• **PANDYA, maulik yogeshbhai**
**Eindhoven (NL)**
• **CHAUDHURY, Sudipta**
**Eindhoven (NL)**
• **BUSSA, Nagaraju**
**Eindhoven (NL)**

(74) Representative: **Philips Intellectual Property & Standards**
**High Tech Campus 52**
**5656 AG Eindhoven (NL)**

(54) **MANAGING A MODEL TRAINED USING A MACHINE LEARNING PROCESS**

(57)    A computer implemented method of managing a first model that was trained using a first machine learning process and is deployed and used to label medical data. The method comprises determining (202) a performance measure for the first model, and if the performance measure is below a threshold performance level, triggering (204) an upgrade process wherein the upgrade process comprises performing further training on the first model to produce an updated first model, wherein the further training is performed using an active learning process wherein training data for the further training is selected from a pool of unlabeled data samples, according to the active learning process, and sent to a labeler to obtain ground truth labels for use in the further training.

Fig. 4

**Description**

TECHNICAL FIELD

Embodiments herein relate to machine learning and models trained using machine learning processes. Particularly but non-exclusively, embodiments herein relate to managing deployed models that are used in medical applications to label medical data.

BACKGROUND

This disclosure lies in the area of machine learning (ML) models and managing deployed machine learning models used in the medical domain. A common saying in the research community is that "Artificial Intelligence models are only as good as the data". This is because building and deploying reliable ML systems is dependent on there being a sufficient quantity of high quality training data. This is especially true in critical applications such as in the medical domain, where high quality (e.g. reliable) ML systems are needed, thus requiring the highest qualities and quantities of data. Most medical ML systems that are developed using Machine Learning techniques are based on supervised learning approaches where the training data is annotated with annotations that improve its quality/trainability by providing supporting information. This also steers the model learning process towards the objective of the model. However, obtaining labels for data to perform supervised learning is an expensive and non-trivial task. There have been many services dedicated to obtaining annotations for data such as amazon mechanical turks, rapid workers and sama source, some of which use crowd sourcing to obtain data labels (see paper by Zhao, Sukthankar & Sukthankar (2011) entitled "Incremental relabeling for active learning with noisy crowdsourced annotations"; IEEE third international conference on privacy, security, risk and trust and 2011 IEEE third international conference on social computing, pages 728-733, 2011) . These services are costly and sometimes impractical. For example, in the medical domain, annotations cannot be crowd sourced for safety reasons. Hence, the questions "which data should be annotated?" and "how much data should be annotated?" are still outstanding in this field.

SUMMARY

As described above, it is often difficult to provide sufficient volumes and quality of annotated training data for training machine learning models for use in medical products and medical decision making. Obtaining annotations in the medical domain can be costly as annotations require specialized skill. Furthermore, after training, such models may need to be updated and this can be difficult, particularly if the model has to be accredited for use before deployment.

Many medical ML models that have been researched and developed never get deployed in hospital settings largely due to the inability to upgrade models in a reliable way, whilst keeping up with strict compliance. For example, the United States (US) Food and Drug administration (FDA) approves ML models before they can be deployed in medical products in the United States (see briefing article by The Pew Charitable Trusts entitled: "How FDA Regulates Artificial Intelligence in Medical Products", July 2021). Generally, the FDA considers such models to constitute "Software as a Medical Device" (SaMD). The FDA has expressed its positive views on in-product adaptation, provided that the adaptation is not significant. It is an object of embodiments herein to improve on current methods in order to better facilitate the use of ML models in medical applications.

It is an object of some embodiments herein to improve upon this situation in order to facilitate the use of high-quality machine learning models in medical products.

Thus, according to a first aspect, there is a computer implemented method of managing a first model that was trained using a first machine learning process and is deployed and used to label medical data. The method comprises: i) determining a performance measure for the first model; and ii) if the performance measure indicates a performance below a threshold performance level, triggering an upgrade process wherein the upgrade process comprises performing further training on the first model to produce an updated first model, wherein the further training is performed using an active learning process wherein training data for the further training is selected from a pool of unlabeled data samples, according to the active learning process, and sent to a labeler to obtain ground truth labels for use in the further training.

According to a second aspect, there is a system for managing a first model that was trained using a machine learning process and is deployed and used to label medical data. The system comprises a memory comprising instruction data representing a set of instructions, and a processor configured to communicate with the memory and to execute the set of instructions. The set of instructions, when executed by the processor, cause the processor to: i) determine a performance measure for the first model; and ii) if the performance measure indicates a performance below a threshold performance level, trigger an upgrade process wherein the upgrade process comprises performing further training on the first model to produce an updated first model, wherein the training is performed using an active learning process wherein training data for the further training is selected from a pool of unlabeled data samples, according to the active learning process, and sent to a labeler to obtain ground truth labels for use in the further training.

According to a third aspect there is a computer program product comprising a computer readable medium, the computer readable medium having computer readable code embodied therein, the computer readable code being configured such that, on execution by a suitable computer or processor, the computer or processor is caused to perform the method of the first aspect.

Thus, according to the system and methods herein, a deployed machine learning model is upgraded using active learning (otherwise referred to herein as query learning) when a performance level indicates a performance below a threshold performance level. In this way, training data is selectively sampled, according to the query learning process, in order to obtain training data that is most likely to improve the quality of the model. In this way the amount of training data that is required can be reduced, thus obtaining good quality improvements, with the minimal labelling effort of the expert labeler.

BRIEF DESCRIPTION OF THE DRAWINGS

For a better understanding and to show more clearly how embodiments herein may be carried into effect, reference will now be made, by way of example only, to the accompanying drawings, in which:

Fig. 1 shows a system according to some embodiments herein;
Fig. 2 shows a method according to some embodiments herein;
Fig. 3 illustrates an example user feedback form for a deployed ML model;
Fig. 4 shows a method according to some embodiments herein; and
Fig. 5 shows a method according to some embodiments herein.

DETAILED DESCRIPTION

Embodiments herein relate to managing models trained using machine learning processes (otherwise known as machine learning models) after deployment.

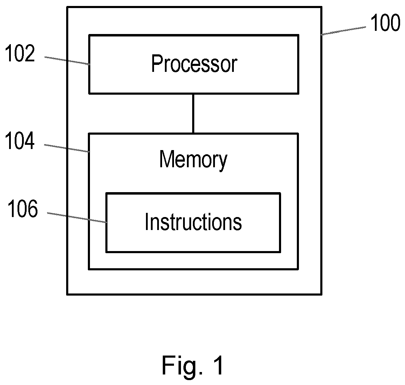

Turning now to Fig. 1, in some embodiments there is a system 100 for managing a first model that was trained using a first machine learning process and is deployed and used to label medical data. The system may form part of specialized equipment, such as specialized medical equipment. More generally, the system may form part of a computer system e.g. such as a laptop, desktop computer or other device. Alternatively, the system 100 may form part of the cloud/a distributed computing arrangement.

The system comprises a memory 104 comprising instruction data representing a set of instructions 106 and a processor 102 configured to communicate with the memory and to execute the set of instructions. Generally, the set of instructions, when executed by the processor, may cause the processor to perform any of the embodiments of the methods 200 as described below. In some implementations, the set of instructions can comprise a plurality of software and/or hardware modules that are each configured to perform, or are for performing, individual or multiple steps of the method described herein.

More specifically, as will be described in more detail below, the set of instructions 106, when executed by the processor, cause the processor to: i) determine a performance measure for the first model; and ii) if the performance measure indicates a performance below a threshold performance level, trigger an upgrade process wherein the upgrade process comprises performing further training on the first model to produce an updated first model, wherein the training is performed using an active learning process wherein training data for the further training is selected from a pool of unlabeled data samples, according to the active learning process, and sent to a labeler to obtain ground truth labels for use in the further training.

The processor 102 can comprise one or more processors, processing units, multi-core processors or modules that are configured or programmed to control the system 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the system 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the system 100. For example, one or more memories 104 may be part of another device. Memory 104 can be used to store the first model, input and output parameters of the first model, and any other information and/or data received, calculated or determined by the processor 102 of the system 100 or from any interfaces, memories or devices that are external to the system 100. The processor 102 may be configured to control the memory 104 to store the first model, the input and output parameters of the first model, and any other information and/or data received, calculated or determined by the processor 102.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

The system may further comprise a user input, such as a keyboard, mouse or other input device that enables a user to interact with the system, for example, to provide initial input parameters to be used in the method described herein.

It will be appreciated that Fig. 1 only shows the components required to illustrate this aspect of the disclosure and, in a practical implementation, the system 100 may comprise additional components to those shown. For example, the system 100 may comprise a battery or other power supply for powering the system 100, or means for connecting the system 100 to a mains power supply.

In more detail, the skilled person will be familiar with machine learning and models trained using machine learning processes (i.e. machine learning models), but briefly, a machine learning process may comprise a procedure that is run on data to create a machine learning model. The machine learning process comprises procedures and/or instructions through which training data, may be processed or used in a training process to generate a machine learning model. The machine learning process learns from the training data. For example, the process may be used to determine how one set of parameters in the training data (input parameters of the model) are correlated with another set of parameters in the training data (output parameters of the model). The machine learning process may be used to fit the model to the training data. Machine learning processes can be described using math, such as linear algebra, and/or pseudocode, and the efficiency of a machine learning process can be analyzed and quantized. There are many machine learning processes, such as e.g. algorithms for classification, such as k-nearest neighbors, algorithms for regression, such as linear regression or logistic regression, and algorithms for clustering, such as k-means. Further examples of machine learning models are Decision Tree models and Artificial Neural Network models.

The first model (which may be otherwise referred to as the first machine learning model), may comprise both data and procedures for how to use the data to e.g. make the predictions described herein. The first model is what is output from the machine learning (e.g. training) process, e.g. a collection of rules or data processing steps that can be performed on the input data in order to produce the output. As such, the model may comprise e.g. rules, numbers, and any other algorithm-specific data structures or architecture required to e.g. make predictions.

Different types of models take different forms. Some examples of machine learning processes and models that may be used herein include, but are not limited to: linear regression processes that produce models comprising a vector of coefficients (data) the values of which are learnt through training; decision tree processes that produce models comprising trees of if/then statements (e.g. rules) comprising learnt values; and neural network models comprising a graph structure with vectors or matrices of weights and biases with specific values, the values of which are learnt using machine learning processes such as backpropagation and gradient descent.

Generally, the first model may be any type of classification or regression model and the first machine learning process may be any supervised or semi-supervised machine learning process. The first model may generally be any type of model that is trained using a training process that uses annotated training data.

For example, the first model may be a decision tree or a random forest-based classifier, see papers: Quinlan (1986) entitled: "Induction of decision trees" Machine Learning volume 1, pages 81-106 (1986); and Breiman (2001) entitled "Random Forests"; Mach Learn 45(1): 5-32. In other embodiments, the first model is a deep neural network, see paper by Schmidhuber (2015) entitled: "Deep learning in neural networks: An overview" Neural Networks Volume 61, January 2015, Pages 85-117. It will be appreciated that the type of model may be dependent on the format of the input parameters (e.g. convolutional neural networks may be more appropriate for taking images as input, for example).

The first model was trained (e.g. before deployment). The first model was trained on labelled data, which may be referred to as "labelled seed data". During redeployment training, labelled seed data is generally split into a Training Pool and a Validation Pool. Training is performed using the Training Pool data according to the first machine learning process and the training is stopped when the first model exhibits good performance on the Validation Pool. Subsequent to the (initial) training, the first model was deployed.

The first model is for use in labelling, (e.g. annotating or making a prediction from) medical data. For example, the first model may be for use in a medical or healthcare program. As an example, the first model may be for annotating medical images, for example, annotating the location and/or nature of lesions in medical images. The first model may be for use in making diagnoses, selecting courses of treatment, analyzing patient risks e.g. of acquiring one or more illnesses or diseases, monitoring medical equipment, or for use in any other process in a medical facility (e.g. hospital, clinic, etc)

The first model may take as input patient data such as demographic data (e.g. age, weight, height, race, sex etc), readings from medical equipment (e.g. readings from blood pressure monitors, heart rate monitors, SpO2 monitors, or any other medical monitor), medical history (e.g. previous conditions, diagnoses, diseases etc), imaging data, e.g. such as x-ray images, ultrasound images, Magnetic Resonance Imaging (MRI) scans, or any other type of patient data.

The first model may provide as output a label, or annotation for the data. In some embodiments, the label may be a prediction, for example, of a condition or diagnosis, or of a treatment that should be given to the patient.

As another example, the first model may be for use in diagnosis of abnormalities in Computed Tomography (CT), Magnetic Resonance Imaging (MRI), or Ultrasound (US) images.

As another example, the first model may be for detecting vital sign events in an Electrocardiogram (ECG), Electroencephalogram (EEG), or Electrooculogram (EOG) data.

For example, the first model may take as input an Electrocardiogram (ECG), Electroencephalogram (EEG), and/or Electrooculogram (EOG) signal. The first model may then provide as output a classification indicating whether (and/or where) the input signal contains a vital sign event. Examples of vital sign events include, but are not limited to: arrhythmia events (e.g. problems/abnormalities in heart rhythm) such as atrial fibrillation, supraventricular tachycardia, bradycardia, heart block and/or ventricular fibrillation.

As another example, the first model may be for use in medical imaging based diagnosis where the first model may be deployed for abnormality detection in medical images. In such an example the first model may take as input a medical image and output a classification. The classification may indicate whether there is an abnormality in the image. In such an example the first model may output the location of the abnormality and/or the nature of the abnormality (e.g. cancer/non-cancer).

It will be appreciated that these are merely examples and that the first model may have been trained to take any combination of input parameters and provide any combination of output parameters, for use in the medical domain.

The first model is deployed, e.g. is made available to medical professionals, or other users. The first model may be deployed, for example, as part of a (medical) computer program or via an Application Programming Interface (API). As such, the first model may be queried by clinicians, medical professions or other users and the outputs of the first model may be used by said clinicians, medical professions and/or other users in medical decision making.

As will be described in more detail below, the first model may have been accredited by a medical body as complying with a medical standard. For example, the first model may be accredited by the FDA or another standards agency.

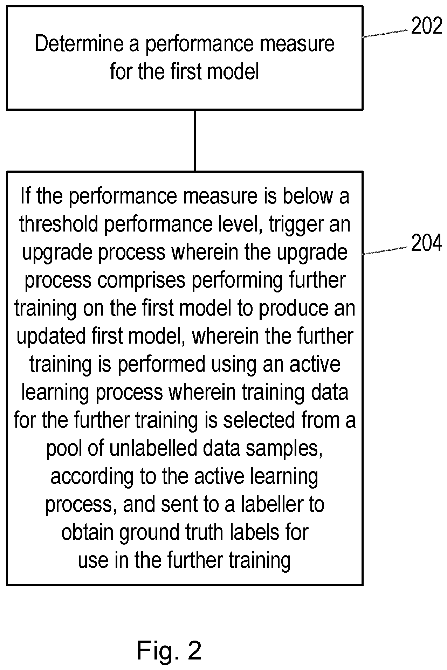

Turning now to Fig. 2 which shows a method 200 according to some embodiments herein. The method 200 is a computer implemented method of managing a first model that was trained using a first machine learning process and is deployed and used to label medical data. The method 200 may be performed by a system such as the system 100 described above with respect to Fig. 1.

Briefly, in a first step 202 the method comprises i) determining a performance measure for the first model. In a second step 204, if the performance measure indicates a performance below a threshold performance level, the method comprises triggering an upgrade process wherein the upgrade process comprises performing further training on the first model to produce an updated first model, wherein the further training is performed using an active learning process wherein training data for the further training is selected from a pool of unlabeled data samples, according to the active learning process, and sent to a labeler to obtain ground truth labels for use in the further training.

In more detail, in step 202, the performance measure can be any measure of how the model is performing. In some examples, the performance measure can reflect the accuracy of the first model, a measure of user satisfaction of the first model or a combination of the accuracy of the first model and a measure of user satisfaction with the model. These are merely examples however and other performance measures could equally be used.

The performance measure may be obtained in any manner. For example, accuracy might be determined using a validation dataset comprising example inputs and ground truth annotations that were not used to train the first model (e.g. previously unseen training data).

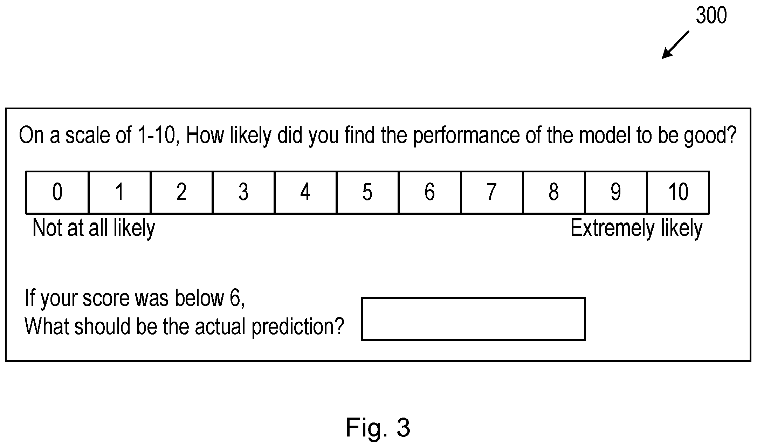

In other examples, a measure of user satisfaction may be obtained from users of the model. For example, via a feedback form 300 such as that illustrated in Fig. 3. User feedback may also be used to obtain correct e.g. ground truth labels for examples where there is low user satisfaction. E.g. the user may be asked to provide the correct label.

A measure of user satisfaction may be based on an average satisfaction. For example, a Satisfaction Score, of the users (who may be Radiologists, r), $SS_r$ calculated as follows:

$$SS_r = \frac{1}{10n} \sum_{i=0}^{n} FS_i$$

Where n is the total number of pieces of feedback received (or held in memory) and $FS_i$ is the ith feedback score.

A measure of accuracy may be any type of accuracy, e.g. such as a loss, or confidence output by the first model when making a prediction.

In some embodiments, a measure of accuracy based on a Blind Validation Score is used. This is the overall accuracy of the model as determined on a Validation Pool of Labelled training data. In embodiments where the first model is a binary prediction model, the blind validation score $VS_b$, is its accuracy given by:

$$VS_b = (TP+TN)/(TP+TN+FP+FN)$$

Where TP is the number of true positives, TN is the number of true negatives, FP is the number of false positives and FN is the number of false negatives. The skilled person will appreciate that this is merely an example, and that the Blind Validation Score may be calculated in various different ways depending on the nature of the outputs of the first model. In other words, the formulation of VSb is task-specific.

Generally, all blind validation is carried out on samples (e.g. input examples) not present in the training dataset. The blind validation can be further enhanced if the samples are obtained from a separate source. For example, a model trained on CT scans obtained using a CT scanner manufactured by a first manufacturer may be validated using CT scans obtained using a different CT scanner manufactured by a second manufacturer. This helps to ensure that the model is generalized.

In some embodiments, a combination of measures may be used. For example, in step 202 the performance measure may be a weighted combination of $SS_r$ and VSb above. E.g. the performance measure, CS may be calculated as:

$$CS = a * SS_r + b * VS_b$$

where *a* and *b* are constants. The values of *a* and *b* can be set in an arbitrary manner, or set so as to give preference to either the user feedback or blind validation score, depending on the preference of a system configuration manager. In other words, the constants *a* and *b* can be set based on the importance given to the user satisfaction and validation performance respectively. As an example, *a* and *b* can each be set to 0.5 so that the maximum value of CS is 1.0.

If the performance measure indicates a performance below a threshold performance level, then in step 204 the method 200 comprises ii) triggering an upgrade process. The upgrade process comprises performing further training on the first model to produce an updated first model. In step 204 the further training is performed using an active learning process wherein training data for the further training is selected from a pool of unlabeled data samples, according to the active learning process, and sent to a labeler to obtain ground truth labels for use in the further training.

The threshold performance level may be set as a system configuration parameter, e.g. according to the preference of a system configuration manager or user. As an example, where the performance measure is the parameter CS described above, and the constants of *a* and *b* are both set at 0.5, then the threshold performance level may be set at 0.8. This is merely an example however, and the threshold performance level may be set at any level deemed appropriate for the particular application.

If the performance measure indicates a performance below the threshold performance level then this triggers the upgrade process whereby further training is performed on the first model using an active learning machine learning process.

The skilled person will be familiar with active learning, otherwise known as query learning. Assuming that there is a huge amount is unlabeled data available free of cost, active learning leverages the help of the first model to determine which unlabeled data needs to be labelled and fed to the first model for retraining. E.g. which examples from the pool of unlabeled data samples should be labelled and used as training data in order to best improve the model. In this way, training data can be labelled selectively, better utilizing the expertise and limited resources of the (human) labeler. Active Learning techniques are reviewed in the paper by Settles, Burr (2009) entitled, "Active Learning Literature Survey", that is published by the University of Wisconsin-Madison Department of Computer Sciences.

As used herein, the labeler is the "Oracle" or annotator of the selected unlabeled data examples in the Active Learning process. The labeler is any expert qualified to label the data. E.g. generally a human expert who is able to provide correct ("ground truth") labels for the selected training data from the pool of unlabeled data samples. In other examples, the labels, or aspects of the labels (e.g. intermediate labels) may be generated in an automated manner.

The pool of unlabeled data samples may be retrieved from a hospital or hospital(s) or any other database of relevant data. For example, it may be retrieved from a picture archiving and communication system (PACS). The pool of unlabeled data samples comprises a plurality of data. Each piece of data or data entry is referred to herein as a data example or instance.

In some embodiments, step 204 may comprise filtering the data. For example, to remove data that cannot be used as training data, e.g. due to incompleteness, high noise levels or incompatibility with the first model.

In some embodiments, the step of performing 204 further training on the first model to produce an updated first model comprises filtering the pool of unlabeled data samples to remove unlabeled data samples that do not have parameters corresponding to the input and/or output parameters of the first model. In other embodiments, step 204 may comprise filtering the pool of unlabeled data samples to remove unlabeled data samples that correspond to a different patient demographic to the patient demographic covered by the first model. In other words, data may be filtered from the pool of unlabeled data samples if it does not fall into the same scope as the first model. Thus, irrelevant data may be filtered from the pool of unlabeled data examples to ensure that remaining data falls within the same scope of the first model in terms of:

Anatomy, e.g. the data describes the correct anatomical features, or body parts. In embodiments where the first model takes imaging data as input, the pool of unlabeled data samples may comprise imaging data that is filtered according to the anatomical features contained in the images.

Modality, e.g. in embodiments where the first model takes imaging data as input, the pool of unlabeled data samples may comprise imaging data that is filtered according to image modality.

Patient Demographics, e.g. age range, or gender, as covered by the first model.

Models/ Original Equipment Manufacturer (OEM). Data arising from different OEMs can have unique characteristics that need to be considered while model building. For example, if a model is developed to work only on a Magnetic Resonance (MR) from a particular manufacturer, with a particular imaging sequence, the dataset might be limited to that particular model, OEM and imaging sequence. If another OEM has similar functionality, then data from their machines may be included as well.

In some embodiments, the step of performing 204 further training on the first model to produce an updated first model comprises filtering the pool of unlabeled data samples to remove unlabeled data samples with high levels of noise.

For example, in embodiments where the first model takes an image as input, noisy images may be removed based on data reconstruction error. For example, the data can be filtered to leave examples where the data reconstruction error is minimum. The data in the pool of unlabeled data samples can be compressed and decompressed using an algorithm such as an Auto Encoder. The reconstruction error is the difference between original data and uncompressed data. This error can be measure using one of the following techniques based on the type of data:

Images: Structural Similarity Index
Signals: Euclidian Norm

The pool of unlabeled data samples that have a high reconstruction error can be filtered out.

Put another way, the unlabeled data samples may be compressed using a compression process, (e.g. such as an autoencoder); the compressed unlabeled data samples may then be reconstructed using a decompression process (e.g. reverse autoencoder). A reconstruction error may then be determined for the reconstructed unlabeled data samples, and the pool of unlabeled data samples may be filtered to remove unlabeled data samples that have a reconstruction error higher than a first reconstruction error threshold. This works on the principle that high quality images with less noise can be more reliably compressed and decompressed than noisy ones.

Filtering can reduce the effort involved in query learning by only selecting high quality, relevant samples for labelling.

Turning back to step 204, training data may be generally selected from the pool of unlabeled data samples (whether filtered or not filtered) in various different manners.

In some embodiments, the active learning process comprises selecting training data from the pool of unlabeled data samples that when passed through the first model, result in outputs that are predicted with low confidence when compared to a threshold confidence level. In other words, the first model may be used to label the data in the pool of unlabeled data samples and data which results in an output of low confidence may be flagged as examples that should be sent to the labeler for labelling and subsequent retraining of the model. The confidence may be described using various different measures, such as for example, posterior probability measures, entropy of the posterior probability, confidence scores etc.

As an example, the entropy of the posterior probability of the first model may be used. When the first model gives a highly uncertain posterior probability when a data instance from the pool of unlabeled data samples is fed, the instance (or example) is chosen for labelling. For example, consider the following sets of posterior probabilities of a two-class classification on two instances

I. Class A: 0.2, Class B: 0.8, entropy = 0.50
II. Class A: 0.55, Class B: 0.45, entropy = 0.688

Certainly, case II is more uncertain and the corresponding instance should be sent for annotation.

In another example, a second model, known as a "side-kick" model to the first model can be used. A side kick model is a model trained using the same training data as the first model, but having different hyper parameters to the first model. The difference in posterior probabilities of both models, given an instance of unlabeled data determines whether that instance need to be labelled. This difference can be calculated using, e.g. Vote entropy, or KL Divergence. For example, data instances in the pool of unlabeled data samples for which the vote entropy is high (e.g. above a predefined threshold), or the Kullback-Leibler (KL) divergence is high (e.g. above a predefined threshold level) can be flagged for labelling. Both of these measures give an indication of whether the side-kick model is deviating from the main-model when fed with certain samples and hence can be flagged for labelling.

Put another way, in some embodiments, the active learning process comprises selecting training data from the pool of unlabeled data samples that, when passed through the first model result in outputs that are different to the outputs of a second model (e.g. a side-kick model) trained using a second machine learning process, the second model having different hyper-parameters to the first model, and wherein the outputs are different by more than a first threshold difference.

In this embodiment, the first model will generally be the same type of model (e.g. neural network, random forest, etc) as the second, sidekick model, but with different hyper-parameters (e.g different numbers of layers, different initialization values of weights/biases). Generally, the second machine learning process will be the same as the first machine learning process (e.g. the first and second models will be trained using the same training process, e.g. gradient descent etc). As above, the difference may be quantified in terms of measures such as vote entropy or KL divergence and the first threshold difference may be set based on these measures dependent on the preference of a system configuration manager.

In some examples, dynamic threshold(s) for the vote entropy and/or KL divergence can be used. Initially the dynamic threshold may be set at an arbitrary value (e.g. an arbitrary threshold of, say 0.3). It may subsequently be increased or decreased based on the number of samples that are flagged for labelling. For example, if fewer samples are required e.g. due to radiologist availability for annotation, then the threshold can be increased so that fewer samples are flagged for annotation. In other words, if the labeler is busy, then the threshold can be increased so that their time is used only for the most critical samples.

The threshold can be automated if the radiologist's allocated time for annotation is known. For example, the threshold can be varied such that the number of flagged responses (Nf) follows the following property:

$$N_f \approx t_{ra} / t_{av}$$

Where $t_{ra}$ is the radiologists time available for an annotation session and $t_{av}$ is the average time to annotate a sample.

Thus, query learning can be used to select appropriate training data with which to perform the further training. The skilled person will appreciate that these are merely examples of how an active learning (e.g. query learning) process might be used to select training data from a pool of unlabeled data examples for use in the further training, see for example the methods described in the paper by Settles, Burr (2009), cited above.

Once selected, as training data, the training data is sent to a labeler to obtain ground truth labels for use in the further training. As noted above, the labeler may be a human expert such as a clinician or medically trained expert. In embodiments where the first model takes image data as input, then the labeler may be a radiologist.

In some cases, multiple clinicians (e.g. radiologists or other medical professionals) might be available for labelling. Thus, in some embodiments, the labeler comprises a plurality of clinicians and the active learning process comprises obtaining annotations from the plurality of clinicians for the selected unlabeled data samples. A weighted combination of the annotations obtained from the plurality of clinicians may thus be used as the ground truth labels for the selected unlabeled data samples.

The plurality of clinicians might have different levels of experience. Thus, the weights for the weighted combination can be set according to a level of experience of each of the plurality of clinicians and/or a level of consistency with which each of the plurality of clinicians provides the annotations.

Annotations can be averaged by considering annotations from more experienced clinicians with higher weights according to:

$$L = c*c1 * L_{R1} + d*c2 * L_{R2}$$

where L is the final label used for the data instance, R1 and R2 are two different clinicians and $L_{R1}$ and $L_{R2}$ are the labels provided by them. The constants c and d are proportions of their experience. c1 and c2 are consistency measures of R1 and R2 respectively. For example, c1 may be set to 1 if the clinician annotates the same image with the same label during two different queries. Clinician experience can be obtained e.g. from a hospital personnel database.

There might be times when the data itself might be of poor quality. This can be determined by sending the data to multiple labelers (e.g. clinicians), and if the data has a low consistency score irrespective of labeler, then the data is considered noisy.

Once the ground truth labels are obtained from the labeler, then further training is performed on the first model, using the selected annotated training data. The further training may be performed in the same manner, e.g. using the same method as the original training performed on the model, e.g. before deployment.

Thus, the method 200 may be used to determining when to upgrade the first model and to determine which data the model should be trained on, so as to only require labelling of specific cases. Thus an upgrade may be performed in a systematic way allowing efficient use of experienced medical practitioners when labelling data. The resulting model is more widely applicable as well as accurate. This reduces the upgrade cost (due to fewer more targeted annotations being required) without compromising the quality.

It will be appreciated that the method 200 may be performed in an iterative (e.g. continuous) manner by repeating steps i) and ii) (e.g. steps 202 and 204). For example, the method 200 may be performed periodically, e.g. at set time intervals, or responsive to a trigger such as upon receiving user feedback.

In further embodiments, compliance testing may be performed on the updated first model. This can be particularly useful in embodiments where the first model is accredited by a body such as the FDA as complying with a medical standard. According to the FDA, a ML model can be upgraded if the upgrade does not severely impact its performance. Thus, some embodiments herein may further involve measuring how much of a change the upgrade is expected to do to the model ("expected model change"). This measure can be used to determine whether to raise a compliance request or raise a decommission request.

In one example, the expected model change may be considered alongside the performance measure described above. For example:

> If the expected model change is small, the first model is upgraded/replaced with the updated first model.
> if the expected model change is high,
> if the performance measure in step 202 is consistently (e.g. over a predefined number of uses of the model, e.g. say 4 uses) above the threshold performance level (e.g. good model performance which would not trigger model upgrade according to step 204) then it is better to go for a compliance request to check the model is still in compliance with the standard.
> if the query learning switch has performance measure in step 202 is consistently (e.g. over a predefined number of uses of the model, e.g. say 4 uses) below the threshold performance level (e.g. poor performance which would trigger model upgrade according to step 204), then the first model may be considered for decommission, or larger scale training, rather than a compliance request.

Expected model change may be calculated from posterior probabilities as follows

The selected training data is used to perform further training on the first model to produce an updated first model.

The updated first model "B" is compared with the (original) first model "A" using a validation data pool (e.g. comprising annotated training examples that were previously unseen, e.g. not used in training the first model or the updated first model) as follows

$$Expected\ Model\ Change\ = \frac{\text{average validation loss B} - \text{average validation loss A}}{\text{average validation loss B} + \text{average validation loss A}}$$

Put another way, the method 200 may further comprise, following the further training, comparing the first model before the further training to the updated first model produced by the further training. If the comparison indicates a difference less than the second threshold difference, deploying the updated first model for use in labelling subsequent medical data. In this way, if the further training results in comparatively small changes to the first model, then the first model may be updated or replaced by the updated first model.

The second threshold difference may be based on an average validation loss, for example. Or any other measure of difference between two models. As described above, in some embodiments, the step of comparing the first model before the further training to the updated first model produced by the further training comprises calculating an average validation loss for the first model $V_{model1}$ before the further training, on a validation dataset; calculating an average validation loss for the updated first model produced by the further training $V_{updatedmodel1}$, on the validation dataset, and calculating an average validation loss between the first model and the updated first model according to: $(V_{updatedmodel1} - V_{model1})/(V_{updatedmodel1} + V_{model1})$.

Thus, the second threshold difference may be a threshold value of $(V_{updatedmodel1} - V_{model1})/(V_{updatedmodel1} + V_{model1})$. If the calculated average validation loss between the first model and the updated first model is above the second threshold difference, then the updated first model may be deployed in place of the (original) first model. If the calculated level of ($V_{updatedmodel1}$ - $V_{model1}$)/($V_{updatedmodel1}$ + $V_{model1}$) is below the Second threshold difference level then a compliance request may be raised, or the model may be decommissioned, as described above.

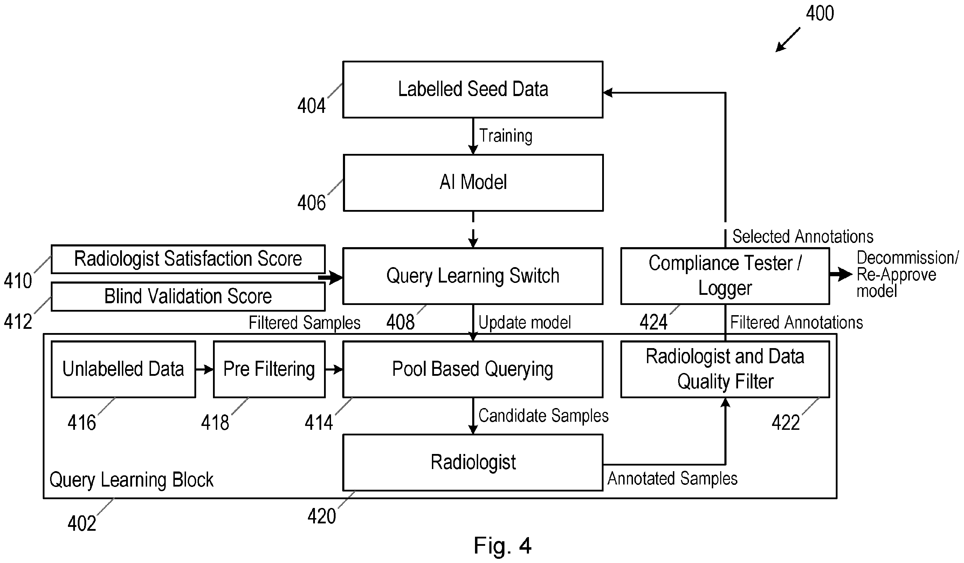

Turning now to Fig. 4, which shows a method 400 of managing a first model that was trained using a first machine learning process and is deployed and used to label medical data, according to an embodiment herein. The method 400 is computer implemented and may be performed by a system such as the system 100 described above.

In step 404, the method 400 comprises training a first model using a training data set comprising labelled seed data. The model is then deployed 406 for use in labelling medical data.

In this embodiment, whilst in use, a performance measure for the first model is determined from user satisfaction scores $SS_r$ 410 and blind validation scores $VS_b$ 412 as described above. The user satisfaction scores may be obtained from e.g. radiologists, clinicians or other users of the first model, e.g. through feedback questionnaires as illustrated in Fig. 3 and obtained as described above with respect to step 202 of the method 200.

The user satisfaction scores $SS_r$ 410 and blind validation scores $VS_b$ 412 are sent to a Query Learning Switch 408 (otherwise known as an "Active Learning Switch") which performs step 202 of the method 200 and determines (e.g. calculates) the performance measure CS as described above, from the user satisfaction scores $SS_r$ 410 and blind validation scores VSb 412. If the performance measure indicates a performance below a threshold performance level, the Query Learning Switch 408 triggers an upgrade process for the first model e.g. triggers step 204 of the method 200 described above). The upgrade process comprises performing further training on the first model to produce an updated first model. The further training is performed using an active learning process that is performed by Query Learning Block 402. As part of the active learning process, training data for the further training is selected from a pool of unlabeled data samples 416, according to the active learning process, and sent to a labeler to obtain ground truth labels for use in the further training.

In this embodiment, the pool of unlabeled data samples 416 are pre-filtered in step 418. Filtering (e.g. to remove samples corresponding to a different patient demographic, to remove noisy samples and/or to remove samples that do not have parameters corresponding to the inputs/outputs of the model) was described above with respect to step 204 of the method 200 and the detail therein will be understood to apply equally to step 418 of the method 400.

In block 414, training data for the further training is selected from the filtered pool of unlabeled data samples 416 using any of the techniques described above with respect to step 204 of the method 200. For example, based on comparison of the output of the first model with the output of a second (side-kick) model or based on posterior probabilities being below a threshold posterior probability.

In block 420 the selected training data is sent to a labeler to obtain labels or annotations. In the embodiment shown in Fig. 4, the labeler is a radiologist 420 but it will be understood that the labeler can be any expert qualified to label the selected training data.

In block 422, if more than one label is obtained, e.g. from more than one radiologist 420, then the labels may be combined, e.g. using a weighted average. The weighted average may be based on experience levels of the labelling radiologists and/or consistency with which they label the data, as described above with respect to step 204. Samples for which the radiologists provide conflicting or different labels may further be filtered out as noisy, or inconclusive.

Further training is then performed on the first model using the selected training data. The updated first model is then sent to a compliance tester/logger module 424 which calculates a measure of difference between the first model and the updated first model according to: (Vupdatedmodell - Vmodell)/ (Vupdatedmodell + Vmodell).

If ($V_{updatedmodel1}$ - $V_{model1}$)/($V_{updatedmodel1}$ + $V_{model1}$) is above a Second threshold difference then the updated first model is sent for compliance testing, otherwise it is deployed in place of the (original) first model.

It will be appreciated that Fig. 4 is an example only and that the functionality described therein may be performed by different computing models/nodes or different combinations of computing models/nodes to those described above.

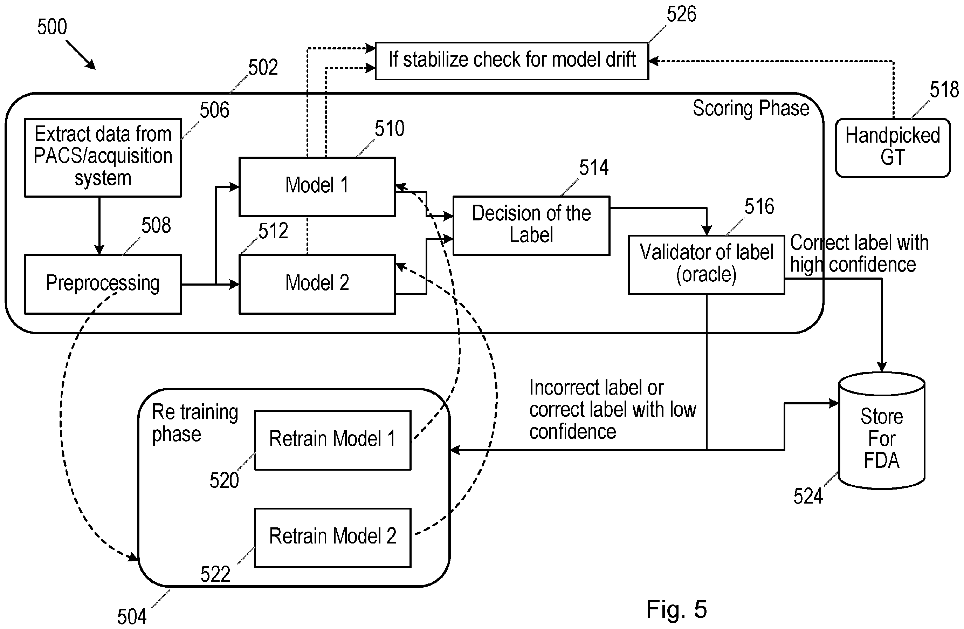

Turning now to Fig. 5 there is an example embodiment showing steps performed by a system 500 for managing a first model. In this example, the first model 510 is for use in stroke detection. For example, the first model may be a deep learning model trained to take as input Computed Tomography (CT) scans of the patient's head and output a classification of whether a hemorrhage (stroke) is detected. The output may be a binary output, e.g. stroke or no-stroke detected. Alternatively, the output may be Hemorrhage / Non Hemorrhage / Can't be determined. These are only examples, and the first model may, alternatively or additionally, produce other outputs, e.g. such as the type of stroke detected. It will further be appreciated that stroke detection is merely an example and that the example illustrated in Fig. 5 may equally be applied to different types of first model for use in labelling other types of medical data.

The steps may comprise steps 502 for assessing the performance of the first model 510 and selecting appropriate training data for use in the active learning process. There may further be steps for re-training the model on the selected training data 504. Steps 502 may be performed as part of step 204 of the method 200 described above.

In this example, in step 204 of the method 200, as part of the active learning process, a pool of unlabeled data samples are extracted 506 from a hospital database such as a picture archiving and communication system (PACS). Preprocessing 508 is performed on the unlabeled data (e.g filtering as described above with respect to step 202 of the method 200, the detail of which will be appreciated to apply equally to the method 500).

The unlabeled data is then fed through a first model "Model 1" 510 and a second model "Model 2" 512. In this example, the second model 512 is a side-kick model to the first model 510, and has different hyper-parameters to the first model 510. The labels produced by each model are compared 514 (for each data instance in the pool of unlabeled data samples) and if the outputs are different by more than a first threshold difference, then that data instance is selected as training data for further training of the first model and sent to a labeler, which in this example is oracle 516, to obtain a label for the data. If the first model labelled the data instance incorrectly then the first model and/or the second model are retrained on the data instance in steps 520 and 522 resulting in an updated first model and/or an updated side-kick model. The updated first model is then deployed in place of the (original) first model 510. The different versions of the first and second models can be stored in a database 524.

It is noted that models have the tendency to drift over time from the original intended performance. Drift may be determined using a handpicked dataset for this purpose. Such a handpicked dataset may be considered a "Golden ground-truth" dataset. The handpicked dataset may be curated with the correct (ground truth, GT) labels and heterogeneity so as to encompass different flavors of data variations. It is intended that on this GT data, the model should perform well, so as to ensure that retraining does not cause deterioration in the model performance. To put this is terms of the software world, GT data can be considered as "Test cases". Thus in step 526, a handpicked dataset 518 can be used, post active learning, to check that the model is stabilized (no new data sample change), and to test for model drift on the stabilized model.

Turning now to other embodiments, in another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer implemented method of managing a first model that was trained using a first machine learning process and is deployed and used to label medical data, the method comprising:

    i) determining a performance measure for the first model; and
    ii) if the performance measure indicates a performance below a threshold performance level, triggering an upgrade process wherein the upgrade process comprises performing further training on the first model to produce an updated first model, wherein the further training is performed using an active learning process wherein training data for the further training is selected from a pool of unlabeled data samples, according to the active learning process, and sent to a labeler to obtain ground truth labels for use in the further training.

2. A method as in claim 1 wherein the performance measure is a measure of accuracy of the first model and/or a measure of user satisfaction of the first model.

3. A method as in claim 1 or 2 wherein the active learning process comprises selecting training data from the pool of unlabeled data samples that:

    when passed through the first model, result in outputs that are predicted with low confidence when compared to a threshold confidence level; or
    when passed through the first model result in outputs that are different to the outputs of a second model trained using a second machine learning process, the second model having different hyper-parameters to the first model, and wherein the outputs are different by more than a first threshold difference.

4. A method as in any one of the preceding claims wherein the step of performing further training on the first model to produce an updated first model comprises:

    compressing the unlabeled data samples using a compression process;
    reconstructing the compressed unlabeled data samples using a decompression process;
    determining a reconstruction error for the reconstructed unlabeled data samples; and
    filtering the pool of unlabeled data samples to remove unlabeled data samples that have a reconstruction error higher than a first reconstruction error threshold.

5. A method as in any one of the preceding claims wherein the step of performing further training on the first model to produce an updated first model comprises:

    filtering the pool of unlabeled data samples to remove unlabeled data samples that do not have parameters corresponding to the input and/or output parameters of the first model; and/or
    filtering the pool of unlabeled data samples to remove unlabeled data samples that correspond to a different patient demographic to the patient demographic covered by the first model.

6. A method as in any one of the preceding claims wherein the labeler comprises a plurality of clinicians and the active learning process comprises:

    obtaining annotations from the plurality of clinicians for the selected unlabeled data samples; and
    using a weighted combination of the annotations obtained from the plurality of clinicians as the ground truth labels for the selected unlabeled data samples.

7. A method as in claim 6 wherein the weights for the weighted combination are set according to a level of experience of each of the plurality of clinicians and/or a level of consistency with which each of the plurality of clinicians provides the annotations.

8. A method as in any one of the preceding claims wherein the first model is accredited by a medical body as complying with a medical standard.

9. A method as in claim 8 comprising:

    following the further training, comparing the first model as it was before the further training to the updated first model produced by the further training; and
    if the comparison indicates a difference greater than a second threshold difference, flagging the updated first model for compliance testing against the medical standard.

10. A method as in claim 9 further comprising:

    following the further training, comparing the first model before the further training to the updated first model produced by the further training; and

if the comparison indicates a difference less than the second threshold difference, deploying the updated first model for use in labelling subsequent medical data.

11. A method as in claim 9 or 10 wherein the second threshold difference is based on an average validation loss and wherein the step of comparing the first model before the further training to the updated first model produced by the further training comprises:

calculating an average validation loss $V_{model1}$ for the first model before the further training, on a validation dataset;

calculating an average validation loss $V_{updatedmodel1}$ for the updated first model produced by the further training, on the validation dataset;

calculating an average validation loss between the first model and the updated first model according to: $(V_{updatedmodel1} - Vmodel1)/ (V_{updatedmodel1} + Vmodel1)$.

12. A method as in any one of the preceding claims further comprising:

repeating steps i) and ii) in an iterative manner.

13. A method as in any one of the preceding claims wherein the first model is for use in diagnosing abnormalities in Computed Tomography, Magnetic Resonance Imaging, or Ultrasound images; or

wherein the first model is for use in detecting events in Electrocardiogram, Electroencephalogram, or Electrooculogram measurements.

14. A system for managing a first model that was trained using a machine learning process and is deployed and used to label medical data, the system comprising:

a memory comprising instruction data representing a set of instructions; and

a processor configured to communicate with the memory and to execute the set of instructions, wherein the set of instructions, when executed by the processor, cause the processor to:

i) determine a performance measure for the first model; and

ii) if the performance measure indicates a performance below a threshold performance level, trigger an upgrade process wherein the upgrade process comprises performing further training on the first model to produce an updated first model, wherein the training is performed using an active learning process wherein training data for the further training is selected from a pool of unlabeled data samples, according to the active learning process, and sent to a labeler to obtain ground truth labels for use in the further training.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 13.

Fig. 1

Determine a performance measure
for the first model

202

If the performance measure is below a
threshold performance level, trigger an
upgrade process wherein the upgrade
process comprises performing further
training on the first model to produce an
updated first model, wherein the further
training is performed using an active
learning process wherein training data
for the further training is selected from a
pool of unlabelled data samples,
according to the active learning
process, and sent to a labeller to
obtain ground truth labels for
use in the further training

204

Fig. 2

300

| On a scale of 1-10, How likely did you find the performance of the model to be good? |
| --- |

| 0 | 1 | 2 | 3 | 4 | 5 | 6 | 7 | 8 | 9 | 10 |
| --- | --- | --- | --- | --- | --- | --- | --- | --- | --- | --- |

Not at all likely                                                  Extremely likely

If your score was below 6,
What should be the actual prediction?

Fig. 3

Fig. 4

Fig. 5

500

502

526  If stabilize check for model drift

518  Handpicked GT

Scoring Phase

506  Extract data from PACS/acquisition system

510  Model 1

508  Preprocessing

512  Model 2

514  Decision of the Label

516  Validator of label (oracle)

Correct label with high confidence

Incorrect label or correct label with low confidence

504  Re training phase

520  Retrain Model 1

522  Retrain Model 2

524  Store For FDA

EP 4 174 721 A1

| | Europäisches Patentamt European Patent Office Office européen des brevets | **EUROPEAN SEARCH REPORT** | **Application Number** EP 21 20 5810 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHU QIUYU ET AL: "Active Learning of Pattern Classification Based on PEDCC-Loss", IEEE ACCESS, IEEE, USA, vol. 9, 26 October 2021 (2021-10-26), pages 147626-147633, XP011887534, DOI: 10.1109/ACCESS.2021.3123095 [retrieved on 2021-11-08] * abstract, sections I-III * | 1-15 | INV. G06N3/04 G06N3/08 G06N20/00 G06K9/62 |
| X | YANG LIN ET AL: "Suggestive Annotation: A Deep Active Learning Framework for Biomedical Image Segmentation", 4 September 2017 (2017-09-04), ADVANCES IN BIOMETRICS : INTERNATIONAL CONFERENCE, ICB 2007, SEOUL, KOREA, AUGUST 27 - 29, 2007 ; PROCEEDINGS; [LECTURE NOTES IN COMPUTER SCIENCE; LECT.NOTES COMPUTER], SPRINGER, BERLIN, HEIDELBERG, PAGE(S) 399 - 407, XP047429111, ISBN: 978-3-540-74549-5 [retrieved on 2017-09-04] * sections 1, 2 * | 1-15 | |
| X | KUO WEICHENG ET AL: "Cost-Sensitive Active Learning for Intracranial Hemorrhage Detection", 13 September 2018 (2018-09-13), ADVANCES IN BIOMETRICS : INTERNATIONAL CONFERENCE, ICB 2007, SEOUL, KOREA, AUGUST 27 - 29, 2007 ; PROCEEDINGS; [LECTURE NOTES IN COMPUTER SCIENCE; LECT.NOTES COMPUTER], SPRINGER, BERLIN, HEIDELBERG, PAGE(S) 715 - 723, XP047485436, ISBN: 978-3-540-74549-5 [retrieved on 2018-09-13] * sections 3, 5 * -/-- | 1-15 | TECHNICAL FIELDS SEARCHED (IPC) G06N G06K |

The present search report has been drawn up for all claims

| Place of search | Date of completion of the search | Examiner |
|---|---|---|
| Munich | 28 April 2022 | Cordara, Giovanni |

page 1 of 2

Europäisches
Patentamt

European
Patent Office

Office européen
des brevets

**Application Number**

**EP 21 20 5810**

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN XIN ET AL: "Sample Balancing for Deep Learning-Based Visual Recognition", IEEE TRANSACTIONS ON NEURAL NETWORKS AND LEARNING SYSTEMS, IEEE, USA, vol. 31, no. 10, 1 October 2020 (2020-10-01), pages 3962-3976, XP011812762, ISSN: 2162-237X, DOI: 10.1109/TNNLS.2019.2947789 [retrieved on 2020-10-05] * section 3 * ----- | 1-15 | |

TECHNICAL FIELDS
SEARCHED      (IPC)

The present search report has been drawn up for all claims

| Place of search | Date of completion of the search | Examiner |
|---|---|---|
| Munich | 28 April 2022 | Cordara, Giovanni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 4 174 721 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHAO ; SUKTHANKAR ; SUKTHANKAR.** Incremental relabeling for active learning with noisy crowdsourced annotations. *IEEE third international conference on privacy, security, risk and trust,* 2011
- *2011 IEEE third international conference on social computing,* 2011, 728-733
- How FDA Regulates Artificial Intelligence in Medical Products. *The Pew Charitable Trusts,* July 2021
- **QUINLAN.** Induction of decision trees. *Machine Learning,* 1986, vol. 1, 81-106
- **BREIMAN.** Random Forests. *Mach Learn,* 2001, vol. 45 (1), 5-32
- **SCHMIDHUBER.** Deep learning in neural networks: An overview. *Neural Networks,* January 2015, vol. 61, 85-117
- **SETTLES, BURR.** Active Learning Literature Survey. University of Wisconsin-Madison Department of Computer Sciences, 2009